Europäisches Patentamt

European Patent Office

Office européen des brevets

(19)

(11) EP 1 224 951 B1

(12) **EUROPEAN PATENT SPECIFICATION**

(45) Date of publication and mention
of the grant of the patent:
**08.11.2006 Bulletin 2006/45**

(51) Int Cl.:
*A61N 1/36* (2006.01)      *A61B 5/103* (2006.01)

(21) Application number: **01305831.8**

(22) Date of filing: **05.07.2001**

(54) **Stair step voltage actuated measurement apparatus**

Durch Spannung gesteuertes Treppenstufen-Messgerät

Appareil de mesure à marche d'escalier à commande par tension

(84) Designated Contracting States:
**AT BE CH CY DE DK ES FI FR GB GR IE IT LI LU
MC NL PT SE TR**

(30) Priority: **19.01.2001 EP 01300493**

(43) Date of publication of application:
**24.07.2002 Bulletin 2002/30**

(73) Proprietor: **Hedgecock, James Lee
Laguna Beach,
California 92651 (US)**

(72) Inventor: **Hedgecock, James Lee
Laguna Beach,
California 92651 (US)**

(74) Representative: **Franks, Robert Benjamin
Franks & Co.
15, Jessops Riverside
Brightside Lane
Sheffield S9 2RX (GB)**

(56) References cited:
**GB-A- 2 123 698          US-A- 4 646 744
US-A- 4 690 145          US-A- 5 020 542
US-A- 5 797 854          US-A- 6 029 090**

EP 1 224 951 B1

**Description**

**Field of the Invention**

The present invention relates to the field of medical science, and particularly although not exclusively to an apparatus for utilizing bio-electric stimulation.

**Background to the Invention**

It is known to perform experimental examination for identifying abnormalities in nerve fibers, by applying an electrical stimulation transcutaneously to a patient.

It is well documented that specific current signal frequencies will selectively stimulate distinct types of nerve fibers, for example 5 Hz selectively stimulates type C nerve fibers, 250 Hz stimulates type A-Delta nerve fibers, and 2000 Hz stimulates type A-Beta nerve fibers. This neuroselectivity of frequencies is exploited by a method employed to measure the lowest level of current intensity a subject can recognize with a transcutaneous electrical stimulus. This method is termed current perception threshold (CPT) diagnosis.

A major problem often encountered during CPT testing is that the cutaneous electrical resistance threshold (CERT), the current signal level required before conduction through skin can occur, may be greater than the current perception threshold, the current at which a patient recognizes that a nerve has been stimulated. In subjects with a greater CERT than CPT the intensity of the diagnosis signal is turned up past the actual CPT without the subject recognizing the stimulus, since the current is not flowing through the skin to the nerve fiber. Once the intensity reaches the CERT and the current begins flowing, then the subject may report a false "high" CPT, which is actually the CERT being reached by the applied signal.

Previously, a "constant current" mechanism of bio-electric stimulation was developed in the 1950s and refined in the early 1980s. This later refinement is disclosed in US patent 4305402 (Katim). Katim's constant current mechanism monitored a sine wave current and regulated it so that once the CERT had been reached the current was maintained automatically so as to sustain the signal intensity at sufficient level to allow a continuous flow of current, even though a manually operated intensity control may be turned to zero. Thereby, on the next measurement in a serial test at a same skin site on a patient, the current is not required again to breach the CERT, and the actual CPT can thereby be more accurately measured.

Katim's constant current mechanism works best with a sinusoidal wave form current. However, a sinusoidal current is quite difficult for a patient to recognize within a very narrow range of intensities. Due to the wide fluctuation in measurements obtained using a sinusoidal voltage, measurements must be averaged before meaningful analysis is possible.

A more recognizable stimulus is that of a modulated square wave signal, and in particular, a modulated square wave current. A square wave form current is used in the prior art Medi-DX 7000 CPT diagnostic device of Neuro-DX Associates Incorporated, 445 Dartmoor Street, Laguna Beach, California, 92651-1430. This device enables location and quantification of nerve pathology caused by injury, metabolic, and toxic exposures, and provides a screening method for patients prior to invasive examinations and procedures are undertaken. Results of up to 95% accuracy in the detection and quantification of nerve pathology are achievable.

The US patent 6029090 (Herbst) discloses a multi-functional electrical stimulation system having a variety of wave forms including a sine, saw-toothed or square wave form. As with similar prior art stimulation devices Herbst's device provides for a wave form that may be customized in terms of pulse widths and pulse repetition rates. Similarly, GB 2123698 (Biostim) discloses a biological electrical stimulator capable of generating a variety of electrical stimulation wave forms being adjustable with regard to amplitude, pulse rate and burst.

Further teachings of the use of a square wave form being an electrical stimulation signal can be found in US 5797854 (Hedgecock), US 4646744 (Zion), US 4690145 (Minnesota Mining) and US 5020542 (Roosmann) however, none of the aforementioned references address the issue of maintaining a flow of current through the skin to the nerve fiber when attempting to measure and determine the CPT in subjects with a greater CERT.

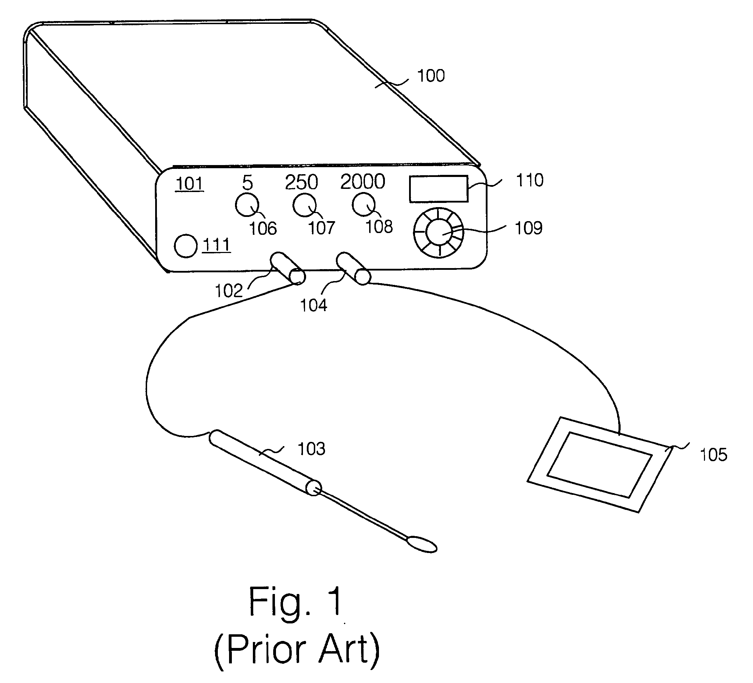

Referring to Fig. 1 herein, there is illustrated schematically in perspective view, the known Medi-DX 7000 current perception threshold diagnostic device. The device comprises a casing 100 containing drive electronics for performing current perception threshold measurements on a patient, the casing having a front panel 101 having a first electrical connector port 102 for connection of a probe device 103; a second electrical connector port 104 for a defuse area electrical contact 105; a set of frequency selector switches 106 - 108 respectively, for selecting test signals having fundamental frequencies corresponding to 5 Hz, 250 Hz and 2 kHz, for testing type C nerves, type A delta nerves, and A- beta nerves respectively; a current intensity control 109 in the form of a rotary dial, having a graduated scale around a circumference of the dial, the rotary dial capable of varying an output current signal in the range 0 to 10 mA between the probe 103 and second electrical contact 105; a liquid crystal display device 110 used to calibrate the current amplitude during manufacture and during after sales service; and an on/off power switch 111.

The usage of the device is known in the art, and is as follows:

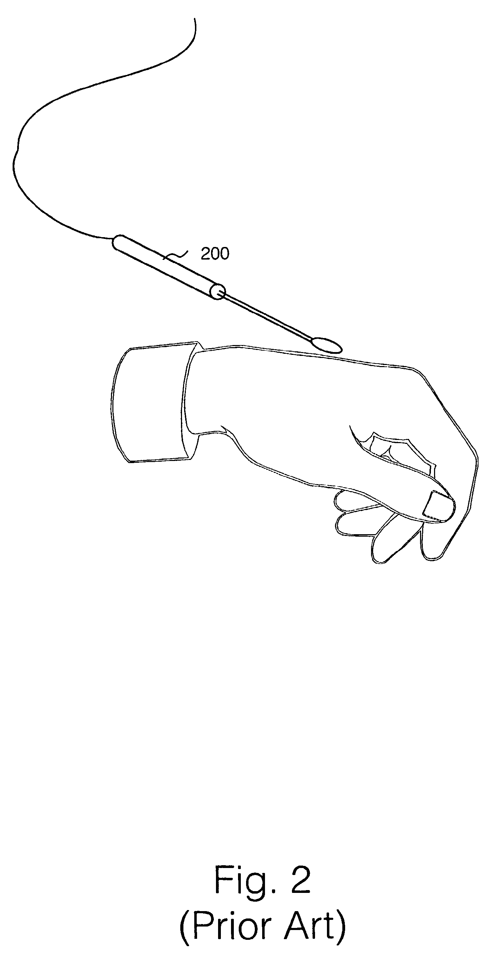

A patient is placed into a relaxed position by a medical personnel. The second electrode contact 105 is placed upon a region of the patient's skin to make electrical contact. The second contact 105 is immersed in saline solution, to improve conductivity between the skin and a wide area contact region of the second contact 105. The probe 103, comprises a gold plated tubular contact 200 capable of receiving a cotton bud 203, which is dipped in saline solution to improve conductivity between the gold contact 200 and a patient's skin as illustrated in Fig. 2 herein. The probe 103 and second electrical contact 105 are placed at various positions around the patient's body, and a square wave electrical signal is passed between the probes through the patient's skin, in order to test various nerves around the patient's body as is known in the art.

To test a particular nerve, the medical operator places the probe and contact at specified positions on the persons body and starting from a zero reading, on the rotary current dial 109 corresponding to zero mean current and gradually increases manually the current by rotating the current control 109, until the patient indicates that a sensation is felt. Due to variations in connection resistance between the cotton bud on the end of the probe 103, and the patient's skin, the medical operator repeats this process 3 or more times for every measurement position, in order to reject spurious readings, and to take a set of readings which are consistent with each other, and which can be used to derive an average reading. Since the medical operator relies upon the patient's perception of sensation due to current, the patient may, either voluntarily or involuntarily, give a misleading indication of when a sensation is felt. For example a patient may, by the intonation of the human medical operator's voice, anticipate when to indicate sensation. Therefore the operator must be careful not to give any indication to the patient of when a sensation could be expected.

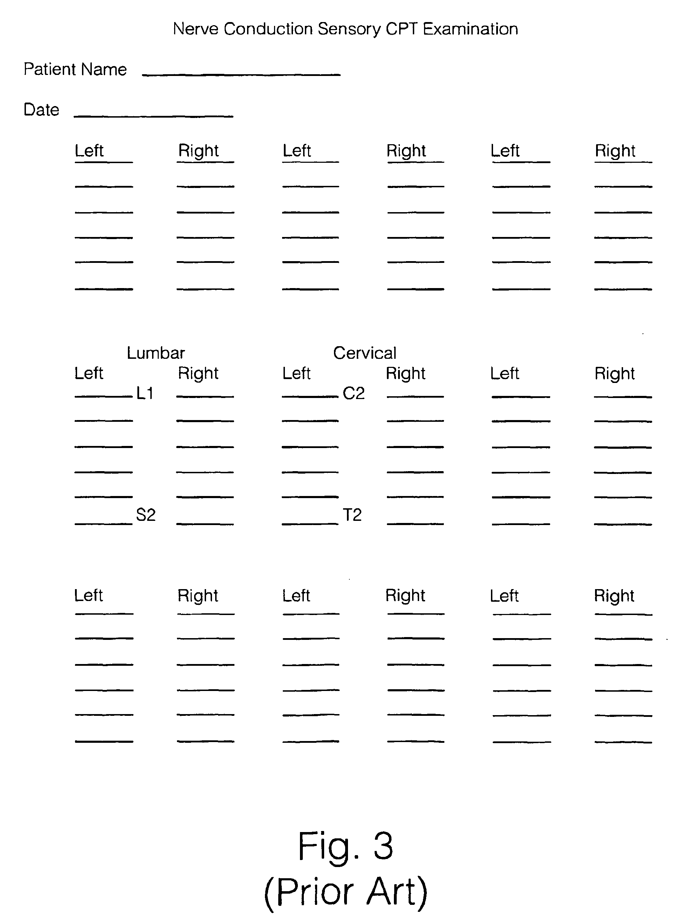

For each nerve tested, the operator manually fills in a record sheet similarly as illustrated in Fig. 3 herein. For example for a cervical test, nerves from the C2 to Thoracic 2 nerve may be tested, on both the left side of the patient's body and the right side of the patient's body and entered onto the record sheet as a current intensity reading on a scale 0 - 100, corresponding to a peak current of 0 to 10 mA. Similarly the medical operator places the probe 103 and second contact 105 on the patient's skin and records readings for the Lumbar L1 to S2 nerves, and other nerve groups as is known in the art.

However, there is a problem in measurement, arising from the electrical conductivity characteristics of a patient's skin. When the probe 103 and second contact 105 are applied to a patient's skin, electrical contact is made via the saline solution, for a current value which has a peak to peak value of a skin conductivity threshold value (CERT), which is determined by the conductive characteristics of the patient's skin. Once the CERT is breached and current is flowing through the skin, provided electrical contact is not lost, and provided the current values does not fall too far below the cutaneous electrical resistance threshold, then current will continue to flow even below the CERT.

However, there is a difficulty in recording readings if the operator returns the rotary current control dial 109 to give a signal too far below the skin's CERT value. Once the current either falls too far below the skin conduction threshold value and current ceases to flow, or if the electrical contact is broken, at a current value below the CERT then the operator must again increase current to exceed the threshold value, before readings can re-commence. Additionally, for nerves which respond to currents near the skin conduction threshold value, obtaining accurate readings is made more difficult.

On some patients, the CERT for some skin sites is higher than the current perception level (CPT). In these cases, measurement is difficult because the operator must first breach the CERT, and reduce the current below the CERT, whilst still maintaining current conduction through the skin, in order to test the patients current perception threshold. If at any time the operator reduces the current dial to zero, current conduction will be lost, similarly if the electrical contact between the probe and the skin is broken, then current ceases and measurements must be re-started at the same site. The current value at which current flow stops is characteristic of each individual patient, and is not a known fixed number.

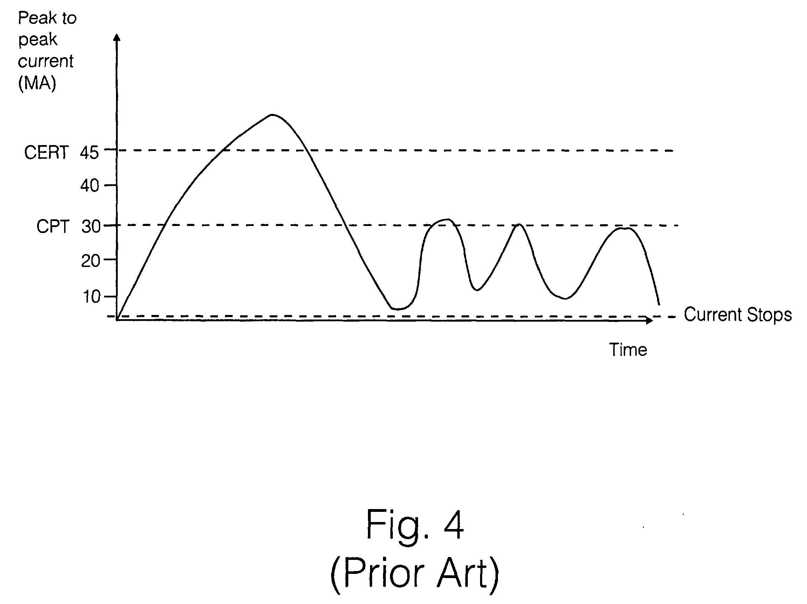

The problem is exemplified, by the plot of peak to peak current versus time shown in Fig. 4 herein which plots an example of the peak to peak current, as controlled by the medical operator, relative to a skin conduction threshold level 401 and a nerve sensitivity threshold level 402.

In this example, the CERT is above the CPT. Initially, the operator turns the current dial from zero up to, for example a reading of 45, at which point the patient indicates that sensation can be felt. This could either be the CERT, or the CPT. At this stage the operator cannot tell which. The operator therefore reduces the current down to a lower reading of 10, and raises the current again slowly. When the dial reads 30, (the level of the CPT) the patient indicates that a sensation is felt, therefore this is likely to be the CPT. However, to verify that, the operator again drops the current to a value of 10, and slowly raises it through the 30 level, at which the patient again indicates a sensation. To verify this a further time, the operator reduces the current back to 10 and raises slowly through 30 at which point the patient verifies a sensation at the current level of 30. In this case, the CERT has a value of 45, and the measured CPT has a value of 30.

The user protocol to deal with this measurement includes:

• At no time can the probe or electrical contact be lifted from the skin sites.
• At no time during the sequence of readings can the intensity be turned to zero (and ideally should not be reduced below a reading of 10 corresponding to 1.0 mA).

- Once the current is turned down, the patient is asked if they continue to feel a stimulus, and if not, then the current is turned up until they feel the stimulus again.
- If an initial higher current measurement is found, then the operator suspects that it is possibly the CERT reading. The operator then turns down the current, but not so far as previously, and asks if the patient continues to feel a stimulus. The operator then turns up the current again until the patient indicates stimulus is felt.
- If the same high reading is noted as previously, then that is the actual CPT and it is a true high reading. Otherwise the initial high reading is a breach of the CERT, and the CPT lies below the initial high reading.

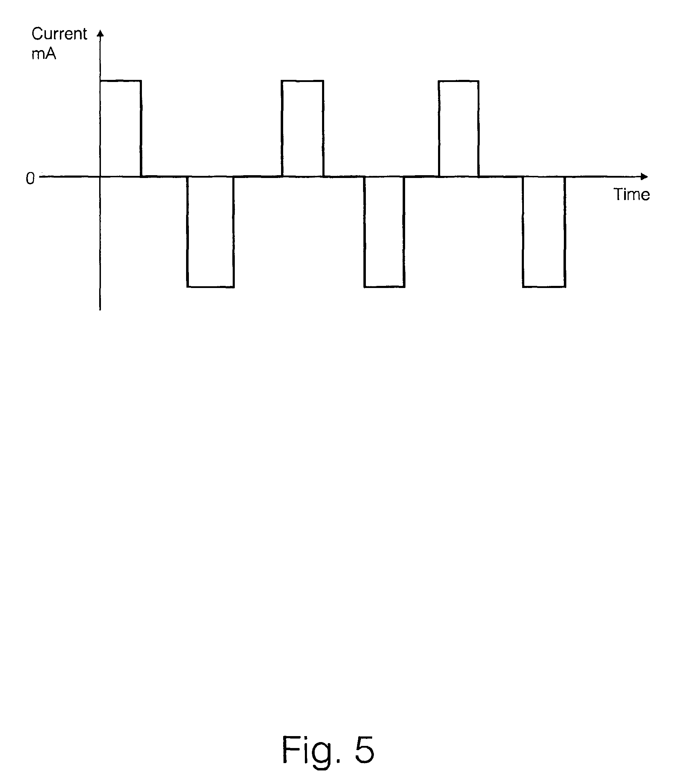

As another example, an operator may initially increase the current to a reading of 45 (4.5 mA) turn down the current to 10 and raise it again for a second reading at 45. The current is then turned down to 20 and a third stimulus reading is recorded at a value of 38. Subsequently the current is turned down to 20 again and a fourth reading is measured at a current of 38 and similarly the fourth or fifth reading also a current is measured at 38. In this case, the CPT is at a value of 38 (3.8 mA) and the CERT (the first two readings) is at a value of 45 (4.5 mA).
If the current is allowed to drop below a critical level at which the current stops (typically between 0 and 10) then current conduction through the skin ceases and the measurement sequence must be started again.
Therefore, to obtain a reliable set of readings, the medical operator must not vary the probe contact to the skin between readings, and ideally should not let the current drop to zero during a set of consecutive readings.
The inventor has recognised various problems associated with the utilisation of bio-electric stimulation for medical diagnosis based on the variation and monitoring of a current. As the aim of the medical diagnosis applied to a patient is to assess the operation of a nerve and in particular a nerve impulse, and given a nerve impulse is caused by, and is directly correlated with voltage the above diagnosis based on current perception threshold provides an indirect measurement of a nerve impulse. A direct measure of a nerve impulse being provided by the monitoring of voltage intensity.
The inventor has further realised that a more recognizable stimulus signal than a sine wave or square wave is that of a modulated square wave form as shown in Fig. 5 herein, in which a duty cycle is controlled so as to produce an interval of zero voltage between an alternating positive and negative square wave cycle. However, it has been noticed that this brief interval of zero voltage can interrupt the current flow, especially when testing with lower frequencies. This interruption necessitates breaching the CERT again, with a false high sensory threshold measurement.

## Summary of the invention

The bio-electric stimulation method as disclosed in Kartim and various of the highlighted prior art, as used to provide an assessment of nerve impulses, assumes, according to Ohm's Law, that the patient's skin has a constant and unchanging electrical resistance. However, this premise is false as, in reality, the skin's resistance is constantly changing and moreover is increasingly varied when current is applied to its surface as in the CPT method, whereby the skin is over-stimulated to release histamine and like substances such that its electrical resistance is substantially altered.
As the method of diagnosis, disclosed herein, involves the measurement of voltage intensity a new terminology is adopted to mirror that of the previously discussed current perception threshold (CPT), this new terminology being Voltage-Actuated Sensory Nerve Conduction Threshold (V-sNCT).
The inventor, through the specific implementations of the present invention, aims to address the above identified problems, namely the inherent inaccuracies involved with a bio-electric stimulation and diagnosis based on current signals and in particular the associated use of bio-electric stimulation using a wave form in the form of a sign or square wave. Based on the fact that the change in a membrane potential, which initiates the nerve impulse, is caused by, and directly correlates with voltage intensity; specific implementations of the present invention aim to provide an apparatus utilising bio-electric stimulation for medical diagnosis based on a direct measurement of the nerve impulse by the measurement of change in membrane potential - measured in volts. The single similarity between the previously discussed CPT diagnosis and the V-sNCT diagnosis according to the use of specific implementations of the present invention, is that both methods use Neuro-selective frequencies to access the type C fibres (5 Hz), A-Delta (250 Hz) and a A-Beta fibres (2 kHz).
Specific implementations of the present invention further aim to correct the problem of interruption of stimulus signal electric flow by use of a unique electrical wave form which has not been employed previously in experimental bio-electrical stimulation, or in any device used for nerve impulse testing. This new wave form is similar to a modulated square wave form signal with a zero interval, however instead of an interval between square waves being zero, the voltage steps down a controllable percentage of a preceding major part of the wave form. A circuit controls this step voltage as a percentage of a major stimulus voltage. Therefore current flow is maintained since current does not completely stop flowing through the patient's skin. The step voltage is maintained at its highest level until a stair step reset operation returns the step voltage to zero before a new site (nerve) is tested. This mechanism allows any major stimulation wave form to be decreased, but not below a predetermined lower step voltage value.

One object according to the specific implementations of the present invention is to provide a single wave form which maintains current conduction through skin during variation of a stimulus voltage signal amplitude.

A second object of specific implementations of the present invention is to improve the usability of a measurement apparatus using bio-electric stimulation.

According to a first aspect of the present invention there is provided apparatus for applying an electric stimulation signal to a patient for performing bio-electrical measurements, said apparatus comprising: a signal generator circuit for generating an electrical stimulation signal, said electrical stimulation signal being alternating, having a positive cycle, a negative cycle and a step signal element wherein said signal generator circuit is configured for generating an electrical stimulation signal having an amplitude that reduces from a peak amplitude to an amplitude of said step signal element; a display device for generating a display of a value of said stimulation signal; a stimulus intensity control for controlling the peak amplitude of said electrical stimulation signal; a frequency selector circuit for selecting a frequency of said electrical stimulation signal; first and second electrical contacts for making contact of said electrical stimulation signal with a skin region of a patient; said apparatus characterised by: a stair step control configured to set the amplitude of said step signal element as a percentage up to 100% of the peak amplitude of said electrical stimulation signal wherein said stair step control is configured for maintaining a flow of said electrical signal through the skin region of the patent and for locking the amplitude of said step signal element to said percentage of the peak amplitude, so that the amplitude of said step signal element changes proportionally with the peak amplitude of said electrical stimulation signal.

**Brief Description of the Drawings**

For a better understanding of the invention and to show how the same may be carried into effect, there will now be described by way of example only, specific embodiments according to the present invention, and methods and processes using the invention, with reference to the accompanying drawings in which:

Fig. 1 illustrates schematically the prior art Medi-DX 7000 current perception threshold (CPT) diagnostic device;

Fig. 2 illustrates schematically placement of a probe contact above a nerve of a patient's left arm;

Fig. 3 illustrates schematically one example a record sheet for recording current intensity readings giving rise to responses of nerve stimulation;

Fig. 4 illustrates schematically a trace of peak to peak current amplitude against time for measurement of a (CPT) level of a patient by a prior art technique;

Fig. 5 illustrates schematically a modulated square wave signal, having an alternating current square wave, interrupted by regions of zero current;

Fig. 6 illustrates schematically a control panel of a novel bio-electric stimulation testing apparatus according to a specific embodiment of the present invention;

Fig. 7 illustrates schematically an electrical stimulation drive wave form generated by the test apparatus of Fig. 6;

Fig. 8 illustrates schematically component signals of the wave form of Fig. 7;

Fig 9 illustrates schematically an example of an output of the bio-electric stimulation test apparatus of Fig. 6, varying over time;

Fig. 10 illustrates schematically a circuit diagram comprising the bio-electric stimulation test apparatus of Fig. 6; and

Fig. 11 illustrates schematically steps for usage of the bio-electric stimulation device for recording measurements of nerve sensitivity.

**Detailed Description of the Best Mode for Carrying Out the Invention**

There will now be described by way of example the best mode contemplated by the inventor for carrying out the invention. In the following description numerous specific details are set forth in order to provide a thorough understanding of the present invention. It will be apparent however, to one skilled in the art, that the present invention may be practiced without limitation to these specific details. In other instances, well known methods and structures have not been described in detail so as not to unnecessarily obscure the present invention.

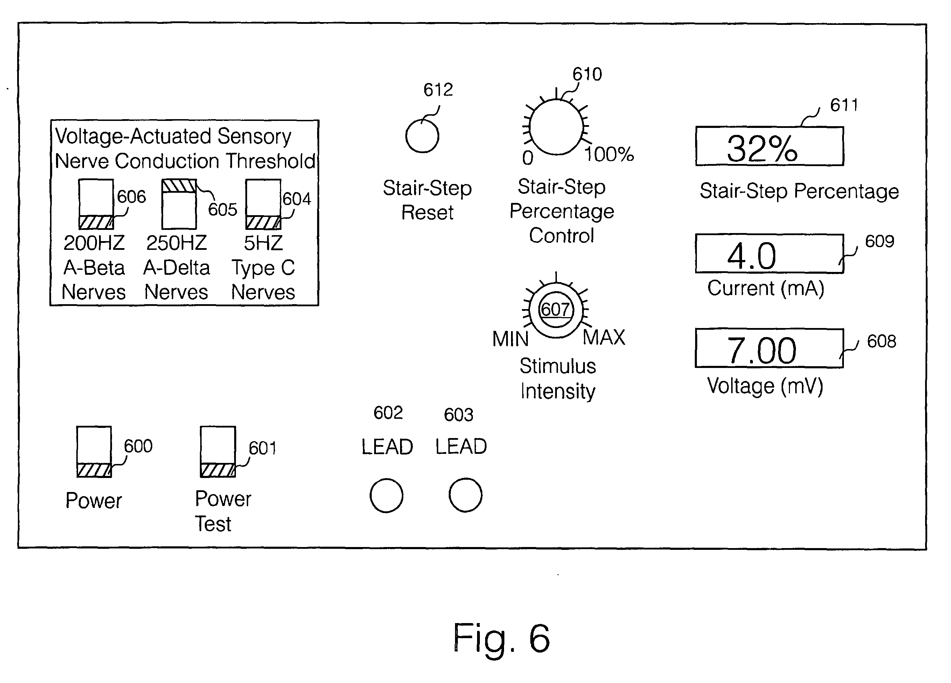

Referring to Fig. 6 herein, there is illustrated a schematically a front pane! of a bio-electric stimulation test apparatus according to a specific implementation of the present invention. The control panel comprises an on/off power switch 600; a power test switch 601, for testing the power supply to the device; an output port 602 for a probe device, as herein described with reference to Fig. 1; an output lead 603 for a second electric contact device, as described herein above; a first frequency switch 604 set to a 5 Hz frequency for testing type C nerves; a second frequency switch 605 set to a 250 Hz frequency for testing type A-Delta nerves; a third frequency switch set to 2 kHz, set for testing type A-Beta nerves; a voltage intensity control dial 607 having a graduated scale from 0 to 100; a first liquid crystal display 608 for displaying a voltage reading in mV between the probe and the second electrical contact; a second liquid crystal display 609 for displaying a current reading in mA; a second rotary voltage intensity control 610 for controlling a percentage of step voltage, the second rotary control knob, optionally having a graduated scale of 0 to 100%; a third liquid crystal display 611, configured to display a step voltage as a percentage of stimulation voltage; and a step voltage reset button 612, for resetting the step voltage to zero.

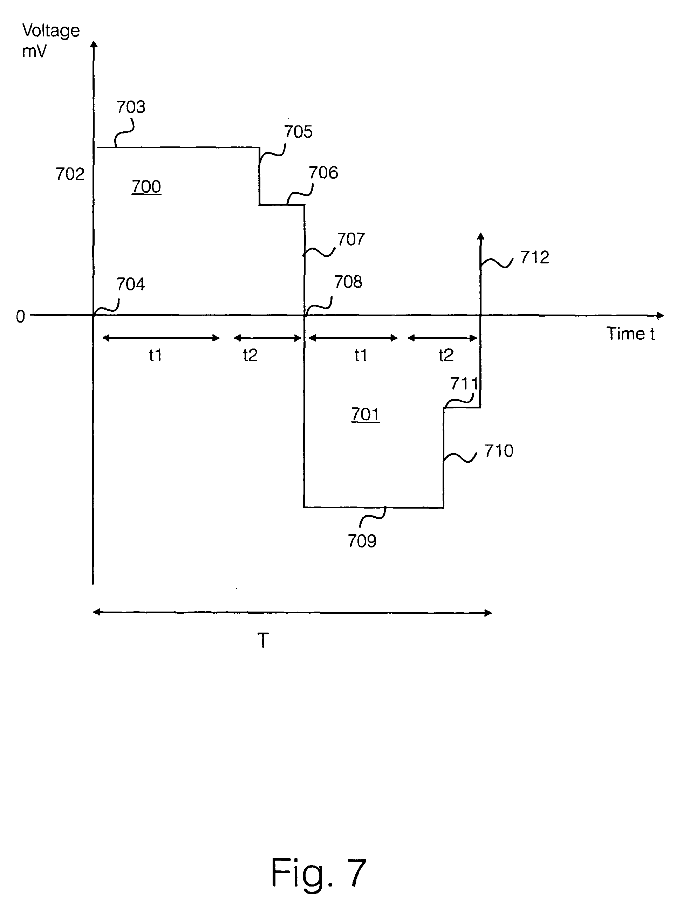

Referring to Fig. 7 herein, there is illustrated a wave form of a drive voltage generated by the stimulation test apparatus between first and second ports 602, 603 for application between a tip of a probe, and a second electrical contact, through a patient's skin. The voltage wave form is characterised by a positive pulse 700 followed by a negative pulse 701, repeating as an alternating voltage. One complete cycle consisting of a positive pulse 700 and a negative pulse 701 has a time duration T. The positive pulse 700 has a positive leading edge 702 followed by a constant stimulation voltage portion 703, followed after a first time period t1 after a positive going zero crossing 704 by a first negative going trailing edge 705, followed by a second constant positive step voltage portion 706 of duration time t2, followed by a second negative going trailing edge 707 crossing through zero voltage at negative going zero point 708, at which the voltage becomes negative; followed by a first negative constant stimulation voltage portion 709 of duration time t1 after the negative going zero crossing 708, followed by a second positive going trailing edge 710, followed by a constant negative step voltage portion 711 of duration t2 after the trailing edge 710 which leads back into a first positive going trailing edge 712 of a next cycle.

The ratio of the time t1 to t2 is variable from t1/(t1+t2) = 0% to t1/(t1+t2)= 100%, by adjustment of a potentiometer device or similar.

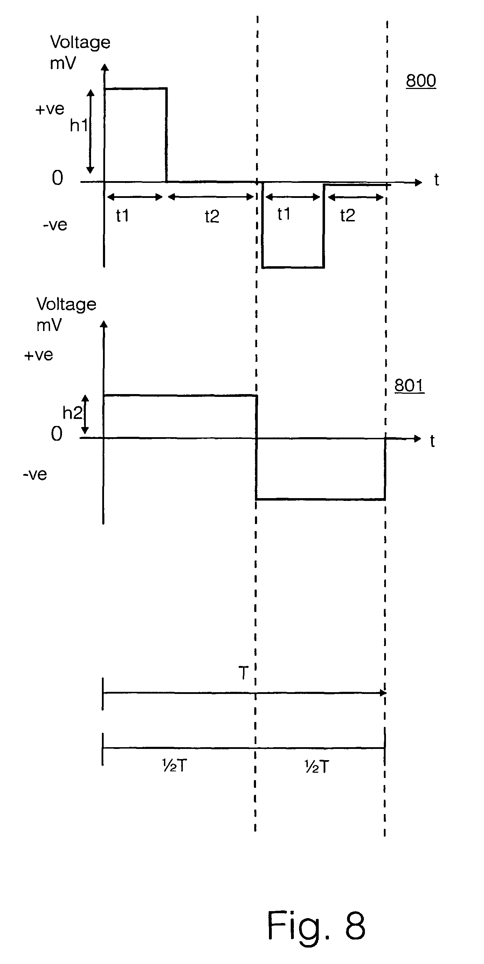

Referring to Fig. 8 herein, the wave form of Fig. 7 can be constructed from a first pulse wave form 800, and a second pulse wave form 801 by adding these two voltage wave forms. First voltage wave form has a first square wave of voltage amplitude of height h1. The second square wave form 801 has voltage amplitude height h2. The two voltage amplitudes h2 and h1 are independently variable, by variation of stair step percentage control knob 610, so that the ratio h2/(h1+h2) is continuously variable from 0% to 100%, with a human operator selecting the percentage.

A minimum value of h2 can be preset by a suitable mechanism, such as by adjustment of a potentiometer provided for that purpose.

If a first pulse wave form 800 is used alone, then particularly at low frequencies such as 5Hz, at some sites on some patients, the zero interval between alternating pulses is long enough to stop the flow of current through the patient's skin. Using the modulated pulse wave form 800 alone, without the step level (step amplitude height H2 = 0% of first square wave amplitude H1), a typical test may be as follows:

- Operator raises the stimulus amplitude h1 to a first reading of 45, at which the patient indicates stimulus.
- Operator turns voltage level down to 10 and takes a second reading raising the voltage to 45, at which the patient indicates stimulus.
- Operator turns down the voltage to 20 and raises again to a level of 45, at which the patient indicates stimulus.
- Operator turns down the voltage to a level of 30 and raises again to 45 at which the patient indicates stimulus.
- Operator turns down the voltage to 40 and raises again to a reading of 45 at which the patient indicates stimulus. The level of 45 could be a CERT, and not the V-sNCT. This could be because the voltage is being interrupted in the period between pulses in the wave form 800, so each time, the CERT must be breached before a patient will indicate sensation.

However, if the step value is applied, at a level of 25% of the stimulus voltage (h2/(h1 + h2) = 0.25, then the test may run as follows:

- Operator increases maximum voltage value to a reading of 45 at which point the patient indicates stimulus.
- Operator turns down the voltage level to 10, but because the step level never goes below 25% of 45 (current conduction is maintained throughout the whole of the duty cycle of the wave form).
- Operator turns up the voltage through a value of 30, at which point the patient indicates stimulus.
- Operator again reduces the dial to 10 (the step voltage maintains the real voltage level), and raises again through level 30 at which point the patient indicates a stimulus.

- The cycle is repeated with the operator reducing the dial to 10 again and raising to 30, with the patient indicating a stimulus at level 30.

In this case, the V-sNCT is 30, and the CERT is 45. Because the voltage is not allowed to drop below a predetermined level of the step value set as 25% of 45, current flow is always maintained, so the CERT is not breached for a second time before the initial reading of 45.

If, in the above sequence, the level of 45 is continuously indicated by the patient, then the step down percentage can be increased, to for example 50%. This may indicate that the step level has been set too low by the operator and voltage flow is still being interrupted. However, in the vast majority of cases, a step voltage level of 25% of the peak stimulus voltage gives a valid reading.

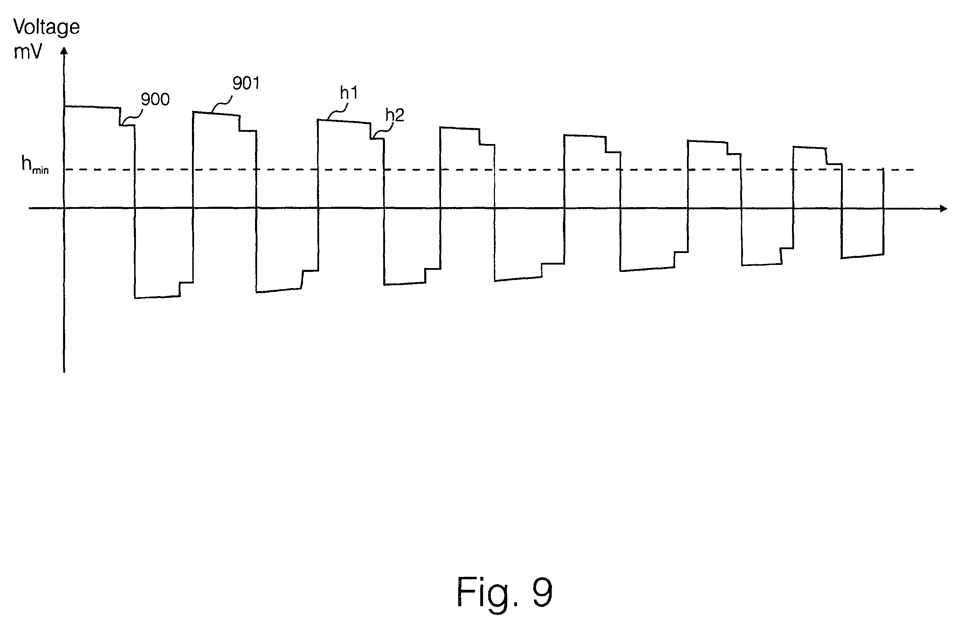

Referring to Fig. 9 herein there is illustrated schematically an example of the wave form changing over time, as a human operator rotates the stimulus voltage intensity dial 607, reducing the peak stimulus voltage.

Over time, as the peak voltage intensity h1 corresponding to the value of first stimulus voltage value 800 is reduced, because the stair step voltage value h2, corresponding to the step voltage square wave form 801 is locked in as a proportion of the stimulus voltage h1, the step portion of the wave form 800 reduces proportionally with the peak stimulus voltage according to the relationship

$$h2 = h1 \times S \text{ for all } h2 > h_{min}$$

$$h2 = h_{min} \text{ for all } h1 \text{ less than or equal to } h_{min}$$

where S is a selectable stair step voltage value as a percentage of stimulus voltage, in the range 0 to 1.0, and $h_{min}$ is a user selectable or factory preset step voltage value with magnitude greater than 0.

Therefore as the voltage is reduced, the maximum stimulus voltage 901 represented by the upper constant portion of the wave form 704 reduces, and whilst the step voltage level 900 is above the preset minimum step value $h_{min}$, the step voltage varies as a proportion of the maximum stimulus voltage. Where the maximum stimulus voltage approaches the preset minimum step voltage $h_{min}$ the peak to peak voltage amplitude of the signal does not fall below $h_{min}$, but is held at the predetermined minimum step voltage value $h_{min}$.

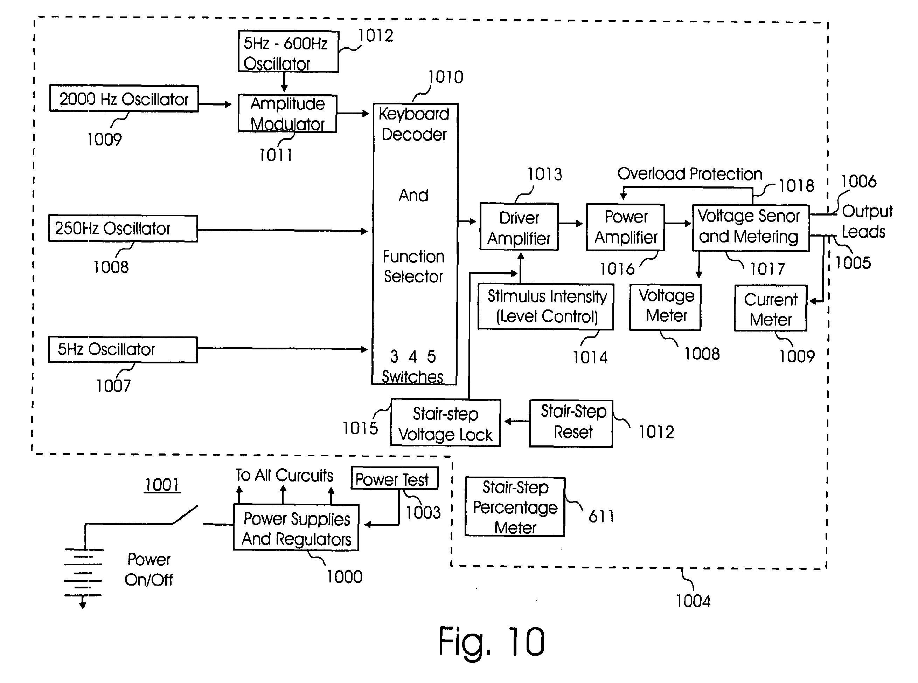

Referring to Fig. 10 herein, there is illustrated schematically a circuit diagram for generating the voltage wave form of Fig. 7. The circuit may be implemented as discrete components, on a circuit board, or as a dedicated chip, for example an application specific integrated circuit (ASIC), comprising analogue and/or digital components, or by a microprocessor, as will be understood by those skilled in the art. The circuit comprises a power supply 1000, being either a battery power supply or a mains voltage derived power supply as is known in the art; a power off/on switch 1001; set of power supplies and regulators providing power to other components; a power test switch 1003 for testing the power supply; and a wave form generation circuit 1004, supplying the wave form to first and second output leads 1005, 1006 respectively corresponding to first and second output ports 602, 603 in Fig.6.

The wave form generator circuit 1004 comprises a plurality of oscillators 1007-1009 set to oscillation frequencies of 5 Hz, 250 Hz, 2000 Hz respectively; a keyboard decoder and function selector 1010 containing frequency switches 604-606 as described herein before; an amplitude modulator 1011 between the 2000 Hz oscillator 1009 and the keyboard decoder and function selector 1010, the amplitude modulator having an input from a 5 Hz to 600 Hz oscillator 1012; a driver amplifier 1013 receiving an input from the keyboard decoder and function selector 1010 which selects a wave form type for amplification; a stimulus voltage intensity level control 1014; a stair step voltage lock circuit 1015 for locking the step voltage as a percentage of the stimulation voltage and setting the minimum step voltage $h_{min}$; a stair step reset circuit, activated by stair step reset switch 612; a driver amplifier 1013 being driven by the stair step voltage lock circuit 1015 and stimulus intensity level control 1014; a power amplifier 1016 receiving an output from the driver amplifier 1013 for amplification; and a voltage sensor and metering device 1017 providing a final output to the output leads 1005, 1006 and applying an overload protection by feedback loop 1018, the voltage sensor and metering stage 1017 having the voltage meter 608 and current meter 609 monitoring the voltage and current at the output leads 1005 1006.

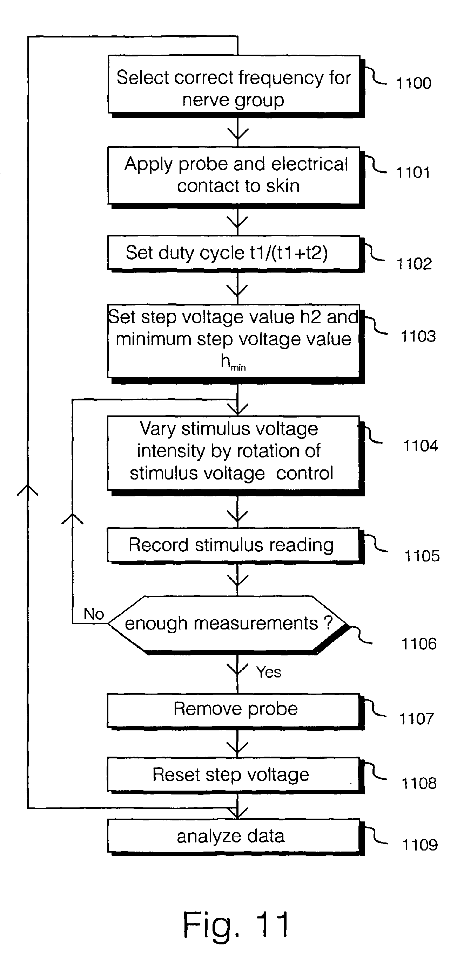

Referring to Fig. 11 herein, there is illustrated schematically a method of use of the stimulation test apparatus. In use, a medical operator applies a probe device and the second electrical contact to a patient's body at the appropriate points, selected for different nerve groups as is known by those skilled in the art. In step 1100, the operator selects a frequency of the signal, either 5Hz, 250Hz or 2kHz in the best mode. In step 1101, the operator applies the probe and electrical contact to the patient's skin. In step 1102, the operator sets the relative time durations of the stimulus voltage and step voltage, within the cycle duration T of the wave form. In the best mode herein favorable results have been found with a ratio t1 as 65% of (t1 + t2), in the 250 Hz frequency range, although good performance is found with t2 in the range 25% to 45% of O.S.T. In step 1103, the operator sets the step voltage value for the set of readings to be taken by rotating the stair step percentage control dial 610 and monitoring visually the reading on the stair step percentage display 611. Steps 1100, 1101 and 1102 need not necessarily be performed in the order shown in Fig. 11. For example the duty cycle, once set may be maintained for different readings, and similarly setting of the step voltage value h2 as a proportion of the stimulation voltage value h1. In step 1104 the operator continues to take the first measurement by varying the stimulus voltage intensity by rotation of the stimulus intensity knob 607, and monitoring the stimulus voltage display 608. When the patient indicates that a stimulus is felt on the nerve, then in step 1105 the operator records manually by writing down the stimulus intensity voltagely displayed, onto a record sheet. The steps 1104, 1105 are repeated until in step 1106, enough measurements are recorded for that measurement site. In step 1107, the probe is removed from the patients skin, and in step 1108 the step voltage is reset to zero, ready for the next set of measurements. In step 1109, the data can be analyzed, having been recorded on a record sheet.

In step 1102, the human operator must first find a suitable value of minimum value stair step voltage value $h_{min}$, by raising the stair step voltage to a point where the voltage display 608 displays an actual voltage flowing between the electrodes. Since the voltage is flowing, the human operator knows that the voltage is above the CERT, and can then reduce the value of the minimum value of stair step voltage h2 to a minimum value, where current still flows. Having established that voltage is flowing through the skin, above the CERT, the human operator proceeds to steps 1104, 1105 to rotate the stimulus voltage intensity dial, varying the height h1 and taking the readings as in step 1105.

In step 1104, because the stair step voltage value h2 is automatically varied as the stimulus voltage intensity value h1 is varied and cannot go below a preset value $h_{min}$, then the human operator can return the stimulus intensity dial below the CERT, without losing electrical conductivity through the skin, which is maintained by the minimum value $h_{min}$ of the stair step voltage value.

It will be appreciated by those skilled in the art that the invention according to the specific implementation as described herein, may be put into effect by the application and variation of current having a stair step modulated square wave form such that current flow in maintained due to a repeating non-zero stepped current being a percentage of the maximum current peak amplitude.

**Claims**

1. Apparatus for applying an electric stimulation signal to a patient for performing bio-electrical measurements, said apparatus comprising:

   a signal generator circuit (1004) for generating an electrical stimulation signal, said electrical stimulation signal being alternating, having a positive cycle, a negative cycle and a step signal element wherein said signal generator circuit is configured for generating an electrical stimulation signal having an amplitude that reduces from a peak amplitude to an amplitude of said step signal element;
   a display device (608, 609, 611) for generating a display of a value of said stimulation signal;
   a stimulus intensity control (607, 1014) for controlling the peak amplitude of said electrical stimulation signal;
   a frequency selector circuit (604-606; 1010) for selecting a frequency of said electrical stimulation signal;
   first and second electrical contacts for making contact of said electrical stimulation signal with a skin region of a patient;

   said apparatus **characterised by**:

   a stair step control (610, 1015) configured to set the amplitude of said step signal element as a percentage up to 100% of the peak amplitude of said electrical stimulation signal wherein said stair step control is configured for maintaining a flow of said electrical signal through the skin region of the patent and for locking the amplitude of said step signal element to said percentage of the peak amplitude, so that the amplitude of said step signal element changes proportionally with the peak amplitude of said electrical stimulation signal.

2. The apparatus as claimed in claim 1, comprising a further display, wherein said apparatus is configured to display a voltage and a current of said electrical stimulation signal and said step signal element.

3. The apparatus as claimed in claim 1 or 2, comprising a step signal element percentage display, for displaying said amplitude of said step signal element relative to said peak amplitude of said stimulation signal.

4. The apparatus as claimed in any one of claims 1 to 3 wherein said stair step control means is configured to control a time duration of said step signal element as a percentage of a total cycle duration of a wave form of said electrical stimulation signal.

5. The apparatus as claimed in claim 4, wherein said stair step control is capable of varying said amplitude of said step element substantially continuously between 0% and 100% of said peak amplitude of said electrical stimulation signal.

6. The apparatus as claimed in any one of claims 1 to 5, further comprising a step value reset button, for resetting said step value after a series of measurements.

7. The apparatus as claimed in any one of claims 1 to 6, wherein said generator circuit is capable of setting a minimum voltage value of said electrical stimulation signal.

8. The apparatus as claimed in any one of claims 1 to 7 wherein said electrical stimulation signal comprises a voltage modulated square wave form, said voltage being stepped down from said peak amplitude during a positive cycle and stepped up from a minimum amplitude during a negative cycle;
wherein said apparatus is configured for direct measurement of a nerve impulse by the measurement of change in membrane potential by variation of the voltage amplitude of said modulated square wave form.

9. The apparatus as claimed in any one of claims 1 to 7 wherein said electrical stimulation signal is **characterized by** a wave form having:

a first leading edge rising to said peak amplitude;
a first trailing edge dropping from said peak amplitude to said amplitude of said step signal element, said amplitude of said step signal element applied for a time duration after said first trailing edge; and
a second trailing edge reducing from said amplitude of said step signal element to a zero amplitude.

**Patentansprüche**

1. Vorrichtung zum Anlegen eines elektrischen Stimulationssignals an einen Patienten, zum Durchführen bioelektrischer Messungen, wobei die Vorrichtung Folgendes umfasst:

eine Signalgeneratorschaltung (1004), zum Erzeugen eines elektrischen Stimulationssignals, wobei das elektrische Stimulationssignal wechselnd ist und eine positive Periode, eine negative Periode und ein Stufensignalelement aufweist, wobei die Signalgeneratorschaltung dazu konfiguriert ist, ein elektrisches Stimulationssignal mit einer Amplitude zu erzeugen, die von einer Spitzenamplitude auf eine Amplitude des Stufensignalelements abfällt;
eine Anzeigevorrichtung (608, 609, 611) zum Generieren einer Anzeige eines Werts des Stimulationssignals;
eine Stimulusintensitätsregelung (607, 1014) zum Regeln der Spitzenamplitude des elektrischen Stimulationssignals;
eine Frequenzauswahlschaltung (604-606; 1010) zum Auswählen einer Frequenz des elektrischen Stimulationssignals;
einen ersten und einen zweiten elektrischen Kontakt zum Herstellen des Kontakts des elektrischen Stimulationssignals mit einer Hautregion eines Patienten;

wobei die Vorrichtung **gekennzeichnet ist durch**:

eine Treppenstufenregelung (610, 1015), die dazu konfiguriert ist, die Amplitude des Stufensignalelements als Prozentsatz von bis zu 100% der Spitzenamplitude des elektrischen Stimulationssignals einzustellen, wobei die Treppenstufenregelung dazu konfiguriert ist, einen Fluss des elektrischen Signals durch die Hautregion des Patienten aufrecht zu erhalten und die Amplitude des Stufensignalelements auf dem Prozentsatz der Spitzenamplitude zu fixieren, so dass sich die Amplitude des Stufensignalelements proportional mit der Spitzenamplitude des elektrischen Stimulationssignals ändert.

2. Vorrichtung nach Anspruch 1, umfassend eine weitere Anzeige, wobei die Vorrichtung dazu konfiguriert ist, eine Spannung und einen Strom des elektrischen Stimulationssignals und des Stufensignalelements anzuzeigen.

**3.** Vorrichtung nach Anspruch 1 oder 2, umfassend eine Stufensignalelement-Prozentsatzanzeige zum Anzeigen der Amplitude des Stufensignalelements relativ zur Maximalamplitude des Stimulationssignals.

**4.** Vorrichtung nach einem der Ansprüche 1 bis 3, wobei das Treppenstufenregelungsmittel dazu konfiguriert ist, eine Zeitdauer des Stufensignalelements als einen Prozentsatz einer Gesamtperiodendauer einer Wellenform des elektrischen Stimulationssignals zu regeln.

**5.** Vorrichtung nach Anspruch 4, wobei die Treppenstufenregelung in der Lage ist, die Amplitude des Stufenelements im Wesentlichen kontinuierlich zwischen 0% und 100% der Maximalamplitude des elektrischen Stimulationssignals zu ändern.

**6.** Vorrichtung nach einem der Ansprüche 1 bis 5, weiter umfassend eine Stufenwert-Rücksetztaste, zum Zurücksetzen des Stufenwerts nach einer Serie von Messungen.

**7.** Vorrichtung nach einem der Ansprüche 1 bis 6, wobei die Generatorschaltung in der Lage ist, einen Mindestspannungswert des elektrischen Stimulationssignals einzustellen.

**8.** Vorrichtung nach einem der Ansprüche 1 bis 7, wobei das elektrische Stimulationssignal eine spannungsmodulierte Rechteckwellenform umfasst, wobei die Spannung während einer positive Periode von der Maximalamplitude abgesenkt wird und während einer negativen Periode von einer Minimalamplitude erhöht wird;
wobei die Vorrichtung für die direkte Messung eines Nervenimpulses durch die Messung der Änderung des Membranpotentials durch Änderung der Spannungsamplitude der modulierten Rechteck-Wellenform konfiguriert ist.

**9.** Vorrichtung nach einem der Ansprüche 1 bis 7, wobei das elektrische Stimulationssignal **gekennzeichnet ist durch** eine Wellenform, die Folgendes aufweist:

eine erste Anstiegsflanke, die auf die Maximalamplitude ansteigt;
eine erste Abfallflanke, die von der Maximalamplitude auf die Amplitude des Stufensignalelements abfällt, wobei die Amplitude des Stufensignalelements während einer Zeitdauer nach der ersten Abfallflanke angelegt wird; und
eine zweite Abfallflanke, die von der Amplitude des Stufensignalelements auf eine Nullamplitude abfällt.

**Revendications**

**1.** Appareil servant à appliquer un signal électrique de stimulation sur un patient en vue d'effectuer des mesures bioélectriques, cet appareil comprenant :

un circuit générateur de signaux (1004) servant à produire un signal électrique de stimulation, ce signal électrique de stimulation étant alternatif, et comportant une période positive, une période négative et un élément du signal en marche d'escalier, **caractérisé en ce que** le circuit générateur de signaux est configuré pour produire un signal électrique de stimulation dont l'amplitude baisse depuis l'amplitude de crête jusqu'à l'amplitude de l'élément du signal en marche d'escalier;
un dispositif d'affichage (608, 609, 611) pour présenter la valeur du signal de stimulation;
un réglage d'intensité de la stimulation (607, 1014) pour contrôler l'amplitude de crête du signal électrique de stimulation;
un circuit sélecteur de fréquence (604-606; 1010) pour sélectionner la fréquence du signal électrique de stimulation;
un premier et un deuxième contacts électriques pour assurer que le signal électrique de stimulation soit mis en contact avec une partie de la peau du patient;

l'appareil étant **caractérisé en ce que**:

un réglage de la marche d'escalier (610, 1015) est configuré pour régler l'amplitude de l'élément du signal en marche d'escalier et la mettre au niveau d'un pourcentage allant jusqu'à 100% de l'amplitude de crête du signal électrique de stimulation, **caractérisé en ce que** le réglage de la marche d'escalier est configuré pour maintenir le passage du signal électrique dans la partie de la peau du patient et pour verrouiller l'amplitude de l'élément du signal en marche d'escalier au pourcentage de l'amplitude de crête, de manière à ce que l'amplitude de l'élément du signal en marche d'escalier varie proportionnellement à l'amplitude de crête du signal électrique de stimulation.

2. Appareil selon la revendication 1, comportant un autre dispositif d'affichage, **caractérisé en ce que** l'appareil est configuré pour afficher la tension et le courant du signal électrique de stimulation et de l'élément du signal en marche d'escalier.

3. Appareil selon la revendication 1 ou la revendication 2, comportant un dispositif d'affichage de l'élément du signal en marche d'escalier en tant que pourcentage, servant à afficher l'amplitude de l'élément du signal en marche d'escalier par rapport à l'amplitude maximum du signal de stimulation.

4. Appareil selon l'une quelconque des revendications 1 à 3, **caractérisé en ce que** le réglage de la marche d'escalier est configuré pour régler la durée de l'élément du signal en marche d'escalier en tant que pourcentage de la durée totale de la période d'une forme d'onde du signal électrique de stimulation.

5. Appareil selon la revendication 4, **caractérisé en ce que** le réglage de la marche d'escalier est capable de faire varier l'amplitude de l'élément du signal en marche d'escalier de manière essentiellement continue entre 0% et 100% de l'amplitude maximum du signal électrique de stimulation.

6. Appareil selon l'une quelconque des revendications 1 à 5, comportant par ailleurs un bouton de remise à zéro de la valeur de la marche d'escalier, servant à remettre à zéro la valeur de la marche d'escalier après une série de mesures.

7. Appareil selon l'une quelconque des revendications 1 à 6, **caractérisé en ce que** le circuit générateur est capable de déterminer une valeur minimum pour la tension du signal électrique de stimulation.

8. Appareil selon l'une quelconque des revendications 1 à 7, **caractérisé en ce que** le signal électrique de stimulation est une forme d'onde carrée modulée en tension, cette tension étant abaissée à partir de son amplitude maximum au cours d'une période positive et élevée à partir de son amplitude minimum au cours d'une période négative; **caractérisé en ce que** l'appareil est configuré pour mesurer directement une impulsion nerveuse en mesurant le changement de potentiel de la membrane provoqué par la variation de l'amplitude de tension de la forme d'onde carrée modulée.

9. Appareil selon l'une quelconque des revendications 1 à 7, **caractérisé en ce que** le signal électrique de stimulation se **caractérise par** une forme d'onde ayant :

un premier front avant dont la valeur augmente jusqu'à l'amplitude maximum;
un premier front arrière dont la valeur baisse depuis l'amplitude maximum jusqu'à l'amplitude de l'élément du signal en marche d'escalier, cette amplitude de l'élément du signal en marche d'escalier étant maintenue pendant une certaine durée après le premier front arrière; et
un deuxième front arrière dont la valeur baisse depuis l'amplitude de l'élément du signal en marche d'escalier jusqu'à une amplitude égale à zéro.

Fig. 1
(Prior Art)

EP 1 224 951 B1

200

Fig. 2
(Prior Art)

Nerve Conduction Sensory CPT Examination

Patient Name  _____

Date  _____

| Left | Right | Left | Right | Left | Right |
|------|-------|------|-------|------|-------|

Lumbar        Cervical

| Left | Right | Left | Right | Left | Right |
|------|-------|------|-------|------|-------|
| ——— L1 | ——— | ——— C2 | ——— | ——— | ——— |
| ——— S2 | ——— | ——— T2 | ——— | ——— | ——— |

| Left | Right | Left | Right | Left | Right |
|------|-------|------|-------|------|-------|

# Fig. 3
# (Prior Art)

Fig. 4
(Prior Art)

EP 1 224 951 B1

Fig. 5

Voltage-Actuated Sensory
Nerve Conduction Threshold

606    200HZ
A-Beta
Nerves

605    250HZ
A-Delta
Nerves

604    5HZ
Type C
Nerves

612
Stair-Step
Reset

610
0        100%
Stair-Step
Percentage
Control

611
32%
Stair-Step Percentage

607
MIN        MAX
Stimulus
Intensity

609
4.0
Current (mA)

608
7.00
Voltage (mV)

600    Power

601    Power
Test

602
LEAD

603
LEAD

Fig. 6

EP 1 224 951 B1

Fig. 7

Fig. 8

Fig. 9

EP 1 224 951 B1

Fig. 10

EP 1 224 951 B1

Select correct frequency for
nerve group — 1100

↓

Apply probe and electrical
contact to skin — 1101

↓

Set duty cycle t1/(t1+t2) — 1102

↓

Set step voltage value h2 and
minimum step voltage value
$h_{min}$ — 1103

↓

Vary stimulus voltage
intensity by rotation of
stimulus voltage control — 1104

↓

Record stimulus reading — 1105

↓

No ⟨ enough measurements ? ⟩ — 1106

↓ Yes

Remove probe — 1107

↓

Reset step voltage — 1108

↓

analyze data — 1109

# Fig. 11